# EUROPÄISCHE PATENTANMELDUNG

(11) **EP 1 584 304 A1**
(43) Veröffentlichungstag der Anmeldung: **12.10.2005**
(21) Anmeldenummer: 04008545.8
(22) Anmeldetag: 08.04.2004
(51) Int. Cl.: A61F 2/06, A61B 17/00

(54) **In eine Ader des Blutkreislaufs einführbares Konduit**

(71) Anmelder: KRAUTH medical KG (GmbH & Co.), 22041 Hamburg (DE); Albert-Ludwigs-Universität Freiburg, 79085 Freiburg (DE)
(72) Erfinder: Höhmann, Reinhard, 22359 Hamburg (DE); Madsen, Tommy, 22399 Hamburg (DE); Schlensak, Christian, 79194 Gundelfingen (DE); Sarai, Koppany, 79241 Ihringen (DE); Torsten, Doenst, 79108 Freiburg (DE)
(74) Vertreter: Glawe, Delfs, Moll

(57) **Zusammenfassung**

Das in eine Ader (4) des Blutkreislaufs einführbare Konduit (7), insbesondere zur Myokardrevaskularisation durch partielle Arterialisierung des Koronarsinus, zeichnet sich dadurch aus, dass es am distalen Ende (13) radial expandierbar ist oder expandierende Elemente trägt, die eine Verankerung des Konduits (7) in der Ader (4) bewirken.

## Beschreibung

Die Erfindung betrifft ein in eine Ader des Blutkreislaufs einführbares Konduit, insbesondere zur Myokardrevaskularisation durch eine partielle Arterialisierung des Koronarsinus.

Die Folgen der koronaren Herzerkrankung mit Einengung oder Verschluss von Herzkranzgefäßen sind mit ca. 30% aller Todesfälle die häufigste Todesursache in allen Industrieländern. In Deutschland sterben jährlich über 340.000 Menschen an den Folgen der koronaren Herzerkrankung. Die aortokoronare Bypass-Operation ist ein etabliertes Therapieverfahren zur Behandlung der koronaren Herzerkrankung, insbesondere wenn mehrere Kranzadern von der Erkrankung betroffen sind. In Deutschland werden pro Jahr etwa 70.000 Bypass-Operationen durchgeführt. Rund 60% der Patienten, die sich zum ersten Mal einer Bypass-Operation unterziehen, sind zwischen 50 und 69 Jahre alt.

Die aorto-koronare Bypass-Operation wird in aller Regel nach Eröffnung des Brustkorbes und Anschluss des Patientens an die Herz-Lungenmaschine durchgeführt. Üblicherweise wird das Herz während des Eingriffes nach Abklemmung der Hauptschlagader für ca. 30 bis 50 Minuten stillgestellt.

Trotz erfolgreicher, primärer chirurgischer Behandlung lässt sich die koronare Herzerkrankung in den meisten Fällen nicht aufhalten und kann ebenfalls auf die angelegten Bypass übergehen. Rund 30% der Bypass sind 15 Jahre nach Anlage wieder verschlossen. Eine erneute, zweite Bypass-Operation ist mit einem deutlich erhöhtem Operationsrisiko und einer Sterblichkeitsrate von etwa 10% verbunden. Das hohe Operationsrisiko einer Zweit-Operation ist begründet durch: 1. Eine mögliche Verletzung des Herzmuskels oder der großen Gefäße bei der Freilegung des in Narbengewebe eingebackenen Herzens. 2. Eine mögliche Verletzung von noch funktionstüchtigem Bypass während der Freilegung des Herzens und daraus resultierendem Herzinfarkt.

Bei vorangeschrittener koronarer Herzerkrankung können die Herzkranzgefäße in ihrem Verlauf so massiv verändert sein, dass das Aufnähen eines Bypass auf die Kranzader technisch nicht möglich ist. Für diese Patientengruppe steht zur Zeit kein etabliertes Therapieverfahren zur Verfügung. Die Herzlaser-Behandlung, die 1994 für Patienten in Deutschland eingeführt wurde, denen mit konventionellen Möglichkeiten (Bypass-OP) nicht mehr geholfen werden konnte, wird nur noch in Ausnahmefällen durchgeführt. Ein möglicher Nutzen für den Patienten konnte bisher nicht nachgewiesen werden.

Für Patienten, die mit konventionellen Operationstechniken nicht mehr behandelt werden können oder bei denen das Operationsrisiko einer Bypass-Operation extrem erhöht ist, wurde das Konzept der Myokardrevaskularisation durch partielle Arterialisierung des Koronarsinus entwickelt.

Bei diesem Verfahren wird ohne den Einsatz der Herz-Lungenmaschine über einen kleinen Hautschnitt an der rechten Halsseite ein Kathetersystem in die Halsvene eingebracht und in die rechte Vorkammer des Herzens vorgeschoben. Das Kathetersystem trägt einen patienteneigenen Venenbypass, oder eine schlauchförmige Prothese aus einem bekannten Material, wie z.B. Dacron, PTFE o.a., d. h. einen Konduit, das bis in den Koronarsinus geführt und dort verankert wird. Danach wird das freie Ende des Konduits durch die rechte Vorkammer in die Halsvene zurückgezogen und über diese ausgeleitet. Anschließend wird das freie ausgeleitete Ende des Konduits mit der Halsschlagader, die direkt neben der Halsvene gelegen ist, verbunden. Somit wird ein Blutstrom von einer zentralen Arterie zum Koronarsinus etabliert, d. h. ein Blutstrom, der der Strömungsrichtung des Koronarsinus entgegengesetzt ist.

Der Zugang zum Koronarsinus bereitet dabei keine speziellen Probleme. Dieser Zugang ist z. B. bei der Enzymmessung im Falle eines Infarkts oder bei der Plazierung von Herzschrittmachersonden bekannt.

Damit das Konduit seine Funktion erfüllen kann, ist es erforderlich, dass sein distales Ende in der koronaren Vene zuverlässig verankert wird. Die Aufgabe der Erfindung besteht in der Schaffung eines Konduits, das in eine Ader des Blutkreislaufs eingeführt werden kann und dann an seinem distalen Ende im Koronarsinus zuverlässig verankert werden kann. Eine weitere Aufgabe der Erfindung besteht in der Schaffung einer Anordnung zum Einbringen eines solchen Konduits.

Die erfindungsgemäße Lösung besteht darin, dass das Konduit am distalen Ende radial expandierbar ist. Es legt sich dabei an die Wand der entsprechenden Ader an, so dass es hier verankert ist. Bei einigen Ausführungsformen legt sich dabei das Konduit an seinem distalen Ende dichtend an die Wand der Ader an, so dass im Falle der eingangs genannten Anwendung in der Gegenrichtung kein Blut strömen kann. In anderen Fällen kann es aber vorteilhaft sein, wenn zumindest eine gewisse Rückströmung in der Gegenrichtung möglich ist. In diesem Falle liegt das distale Ende des Konduits bei anderen Ausführungsformen nicht dichtend an der Wand der Ader an.

Bei einer vorteilhaften Ausführungsform ist am distalen Ende des Konduits im Lumen desselben ein aufweitbarer Stent angeordnet. Bei einer anderen Ausführungsform ist das distale Ende des Konduits außen auf den Konduit so zurückgefaltet, dass eine ringförmige Tasche gebildet wird, in der ein aufweitbarer Stent angeordnet ist.

Der Stent ist dabei vorteilhafterweise durch einen Ballonkatheter aufweitbar. Bei anderen Ausführungsformen ist der Stent selbstexpandierend. Dies kann einerseits dadurch erzielt werden, dass der Stent aus einem Memorymetall hergestellt ist, das zunächst zusammengedrückt ist und dann bei Erwärmung im Körper sich expandiert und dabei seine ursprüngliche Form wieder annimmt. Es ist auch möglich, den Stent zunächst durch eine schlauchförmige Hülse, ein sogenanntes Sheet, zusammengedrückt zu halten, das nach Einbringung des Konduits über den Katheter zurückgezogen wird, so dass der Stent freigegeben wird.

Bei einer anderen vorteilhaften Ausführungsform ist das Konduit am distalen Ende an seiner äußeren Oberfläche mit einer ringförmigen Hülse aus einem Hämostyptikum versehen. Dieses Hämostyptikum quillt auf, wenn es mit Blut in Berührung kommt, und verschließt somit den Raum zwischen Aderwand und Konduitoberfläche.

Bei noch einer anderen Ausführungsform sind im distalen Ende des Konduits ein oder mehrere radial expandierende Drahtelemente angeordnet. Diese können wieder aus Memorymetall bestehen oder aus Federelementen bestehen, die durch ein Sheet zunächst zusammengehalten werden.

Allen diesen Ausführungsformen ist gemeinsam, dass sie das distale Ende des Konduits dicht in der Ader verankern.

Bei den folgenden Ausführungsformen ist dagegen vorgesehen, dass zwar eine Verankerung stattfindet, aber ein Raum zwischen Konduit und Aderwand verbleibt, durch den eine gewisse Rückströmung erfolgen kann.

Bei einer vorteilhaften Ausführungsform sind im distalen Ende mehrere federnde Elemente verankert, deren vorstehende Enden radial expandierbar sind. Die Enden können wieder durch eine auf den Konduit aufgeschobene Hülse (Sheet) zusammengehalten werden, die nach Einbringung des Konduits zurückgezogen wird.

Bei einer anderen Ausführungsform erstrecken sich die federnden Elemente durch das Konduit und sind in demselben vom proximalen Ende her verschiebbar. Es ist in diesem Falle möglich, die federnden Elemente erst aus dem Konduit herauszudrücken, wenn es in die Ader eingebracht ist.

Wenn die federnden Elemente hakenförmig sind, ist eine besonders zuverlässige Befestigung möglich.

Bei einer vorteilhaften Ausführungsform sind die federnden Elemente Spreizfedern.

Bei einer weiteren vorteilhaften Ausführungsform ist das Konduit am distalen Ende an seiner äußeren Oberfläche mit mehreren am Umfang angeordneten Elementen aus einem Hämostyptikum versehen. Dies quillt bei Kontakt mit Blut auf, lässt aber zwischen den einzelnen Elementen Raum für eine gewisse Rückströmung.

Eine Anordnung zum Einbringen eines Konduits der Erfindung zeichnet sich dadurch aus, dass sie einen Führungsdraht, ein Konduit gemäß einer der oben erwähnten Ausführungsformen, einen Katheter, auf den das Konduit aufgeschoben ist, und eine schlauchförmige Hülse (Sheet) aufweist, die auf das Konduit aufgeschoben und nach Einbringung des Konduits nach proximal wenigstens teilweise zurückziehbar ist. Der Katheter kann vorteilhaft an seiner distalen Spitze einen Ballon zur Aufweitung des Konduits und/oder der oben beschriebenen Verankerungselemente haben.

Die Erfindung wird im folgenden anhand von vorteilhaften Ausführungsformen unter Bezugnahme auf die beigefügten Zeichnungen beispielsweise beschrieben. Es zeigen:
- Fig. 1a und 1b: Darstellung des Herzens mit den relevanten Blutgefäßen;
- Fig. 2: in schematischer Darstellung das Prinzip des Einbringens eines Konduits;
- Fig. 3 bis 9: im Querschnitt verschiedene Formen des distalen Endes des Konduits;
- Fig. 10: eine Draufsicht auf das distale Ende des Konduits der Fig. 9;
- Fig. 11 bis 13: Querschnittsansichten weiterer Ausführungsformen;
- Fig. 14: in Draufsicht das distale Ende des Konduits der Fig. 13;
- Fig. 15: eine weitere Ausführungsform im Querschnitt;
- Fig. 16: in Draufsicht das Ende des Konduits der Figur 15; und
- Fig. 17 und 18: weitere Ausführungsformen im Querschnitt.

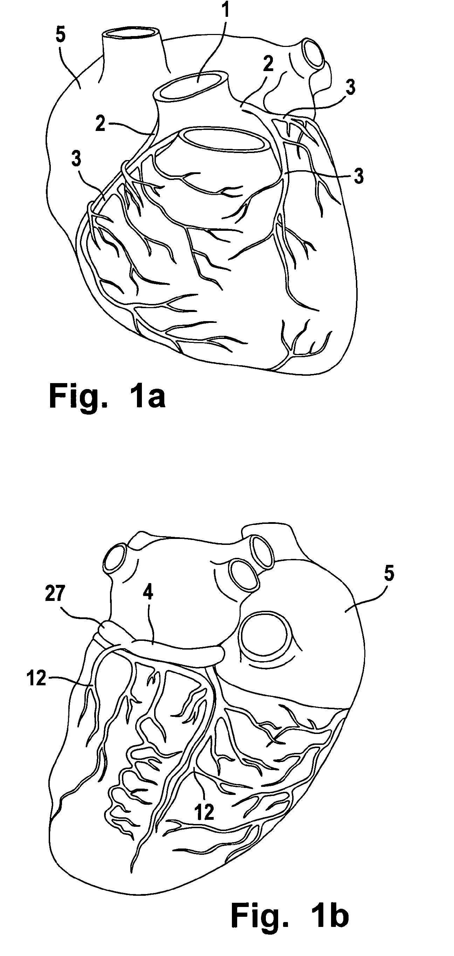

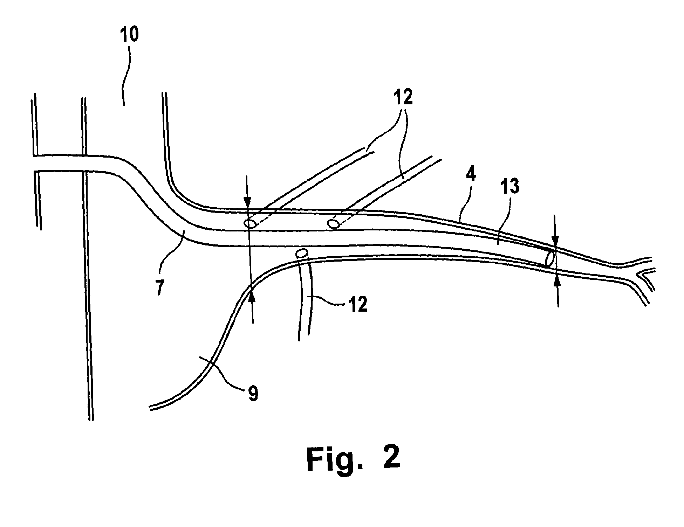

In Fig. 1a und b ist schematisch ein menschliches Herz gezeigt. Aus der Aorta 1 strömt u.a. durch eine Öffnung 2 Blut in die Herzkranzgefäße 3 und versorgt damit die Herzmuskeln. Das zurückfließende Blut kommt aus der Koronarvene 7 und diversen Seitenästen 12, sammelt sich im Koronarsinus 4 und fließt ab in den rechten Vorhof. Bei vorangeschrittener koronarer Herzerkrankung oder bei Rezidivoperationen können die Herzkranzgefäße in Ihrem Verlauf so massiv verändert sein, dass das Aufnähen eines Bypass auf die Kranzader technisch nicht möglich ist. Für diese Patienten ist eine Revaskularisation der Herzkranzgefäße möglich, indem arterielles Blut "von hinten" den Herzkranzgefäßen zugeführt wird, also über den Koronarsinus. Dazu wird, wie dies in Figur 2 schematisch gezeigt ist, ein Konduit 7 durch das Ostium des Koronarsinus in den Koronarsinus 4 eingeführt. Das Konduit 7 erstreckt sich dabei vom Koronarsinus durch den rechten Vorhof 8 und durch die obere Hohlvene 9 in eine große zentrale oder periphere Arterie (in aller Regel die Halsschlagader) 10. Der Übergang vom venösem zum arteriellen Gefäß bereitet dabei keine besonderen Schwierigkeiten und ist übliche chirurgische Praxis, so dass darauf nicht näher eingegangen werden muss. Am distalen Ende 13 muss das Konduit aber im Koronarsinus 4 verankert werden. Diese Verankerung wird man häufig abdichtend vornehmen, da in den Koronarsinus noch kleine Adern 12 führen. In anderen Fällen ist es aber nicht erwünscht, hier eine vollständige Abdichtung zu schaffen, um eine gewisse Rückströmung venösen Blutes zu erhalten.

Verschiedene Arten der Befestigung und/oder Abdichtung des distalen Endes 13 sind in den folgenden Figuren gezeigt.

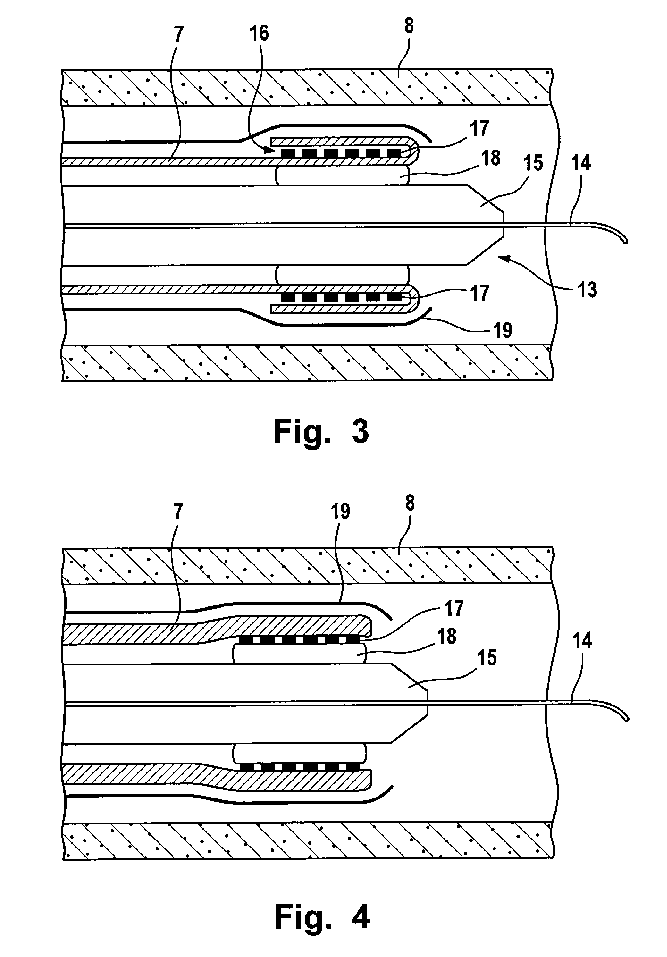

In Figur 3 ist ein Führungsdraht 13 gezeigt, mit dem ein Katheter 14 in den Koronarsinus 4 eingeführt wird. Die Verwendung solcher Führungsdrähte 14 ist allgemein bekannt. Sie werden zunächst in die Ader eingeschoben. Erst anschließend wird dann auf diesen Führungsdraht 14 der Katheter 15 aufgeschoben. Der Katheter trägt das Konduit 7, das bei der Ausführungsform der Figur 3 am distalen Ende 13 umgeschlagen ist, um so eine Tasche 16 zu bilden, in der ein Stent 17 angeordnet ist. Dieser Stent 17 kann mit Hilfe eines Ballons 18, der auf dem Katheter 15 angeordnet ist, erweitert werden, so dass sich das distale Ende 13 des Konduit 7 an die Wand der Vene 8 anlegt. Vorher wird noch eine schlauchförmige Hülse 19, die die genannten Teile umschließt, zurückgezogen. Nachdem der Stent 17 erweitert worden ist, wird dann der Katheter 15 aus dem Konduit 7 entfernt.

Figur 4 zeigt eine ähnliche Ausführungsform, bei der allerdings der Stent 17 im Lumen des Konduits 7 angeordnet ist. In diesem Falle braucht das distale Ende des Konduits 7 nicht umgeschlagen werden.

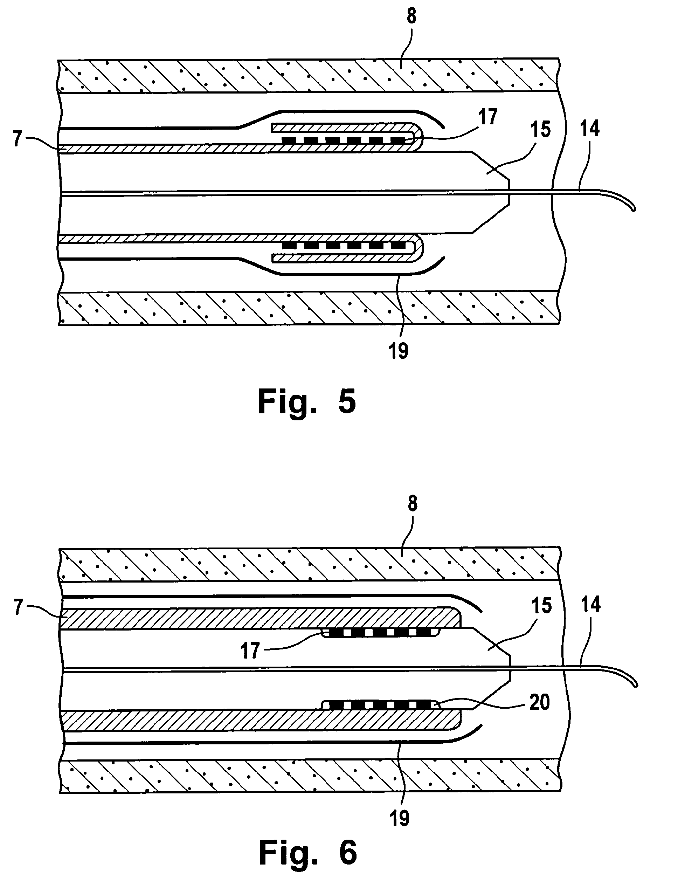

Bei den Ausführungsformen der Figuren 5 und 6 ist der Stent 17 ein selbstexpandierender Stent. Er ist bei der Ausführungsform der Figur 5 in einem umgeschlagenen Ende des Konduits 7, bei der Ausführungsform der Figur 6 innerhalb des Konduits 7 vorgesehen. Bei der Ausführungsform der Figur 6 ist zu diesem Zweck der Katheter 15 mit einer ringförmigen Ausnehmung 20 versehen, in der der Stent 17 vor seiner Expansion untergebracht ist. Die Expansion des Stents 17 kann dabei dadurch bewirkt werden, dass er aus einem Memorymetall ist und sich bei Erwärmung auf Körpertemperatur ausdehnend in seine anfängliche Gestalt zurückverformt. Der Stent könnte auch federnd ausgebildet sein und zunächst durch die schlauchförmige Hülse 19 zusammengehalten werden und dann expandieren, wenn diese entfernt wird.

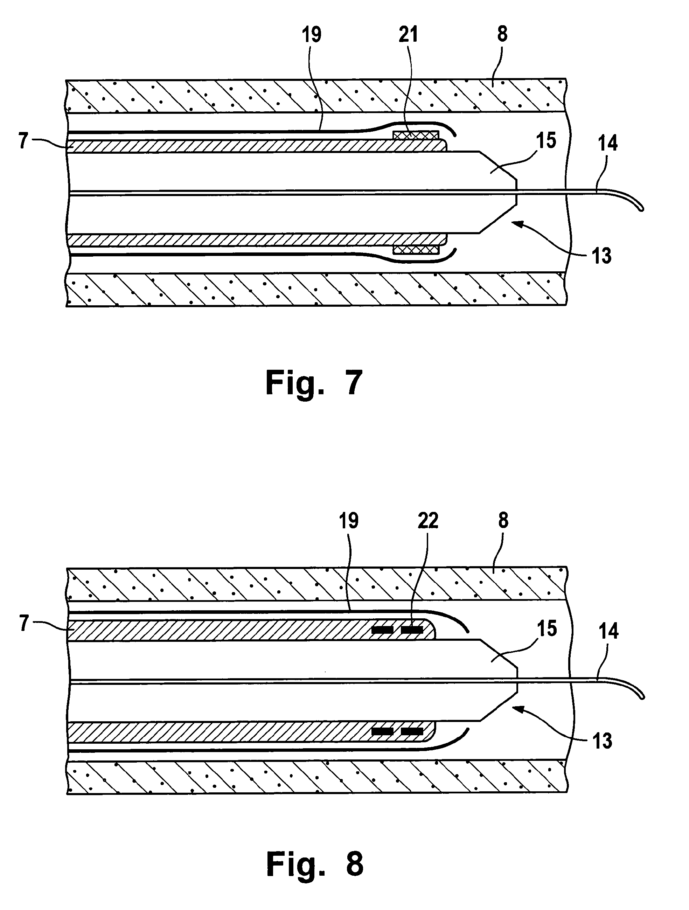

Bei der Ausführungsform der Figur 7 ist am distalen Ende 13 des Konduits 7 ein ringförmiges Element aus einem Hämostyptikum 21 vorgesehen, das sich nach Entfernen der schlauchförmigen Hülse 19 und bei Kontakt mit Blut ausdehnt bzw. aufquillt, um so das Konduit 7 in der Ader 8 dichtend zu verankern.

Bei der Ausführungsform der Figur 8 ist im distalen Ende 13 des Konduits 7 ein drahtförmiges Element oder mehrere drahtförmige Elemente vorgesehen, die sich ebenfalls ausdehnen und das Konduit dichtend an die Wand der Ader 8 anlegen. Die Ausdehnung kann dabei wieder aufgrund der Tatsache erfolgen, dass die Drähte aus einem Memorymetall oder aus Federelementen sind und zunächst durch die Hülse 19 zurückgehalten werden.

Alle vorgenannten Ausführungsformen dichten das distale Ende 13 des Konduits 7 gegen die Wand der Ader 8 ab. Dies ist bei den folgenden Ausführungsformen nicht der Fall.

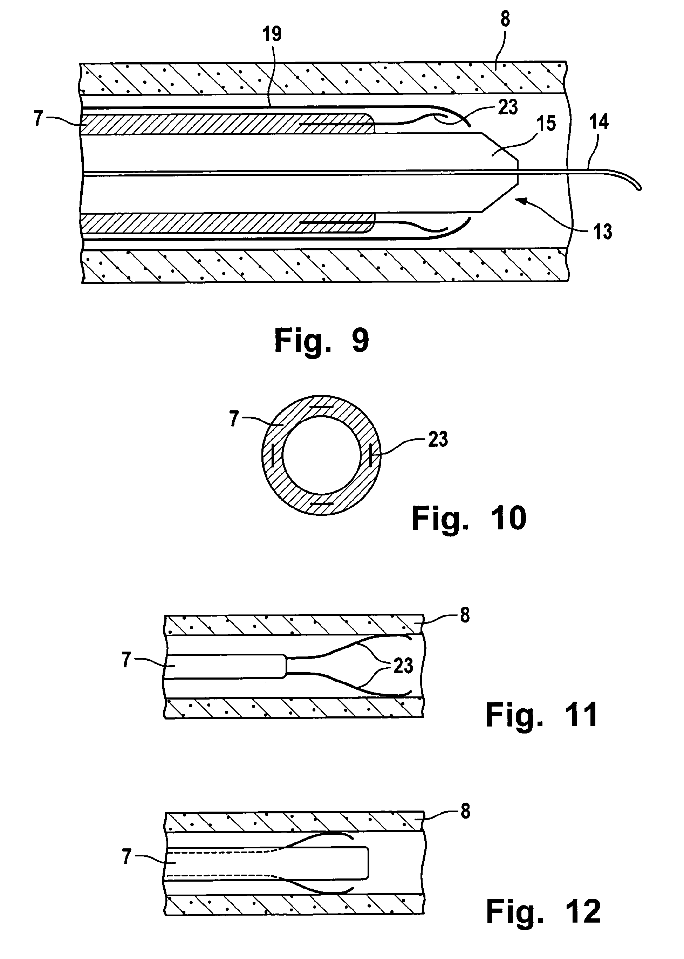

Bei der Ausführungsform der Figur 9 sind im distalen Ende 13 des Konduits 7 Federelemente 23 verankert, die sich nach Entfernen der schlauchförmigen Hülse 19 nach außen an die Wand der Ader 8 anlegen. Es sind mehrere solcher Federelemente 23 vorgesehen, wie dies in der Draufsicht der Figur 10 gezeigt ist, die zwischen sich Räume freilassen, so dass zwischen Konduit 7 und Wand der Ader 8 eine Durchströmmöglichkeit für Blut besteht.

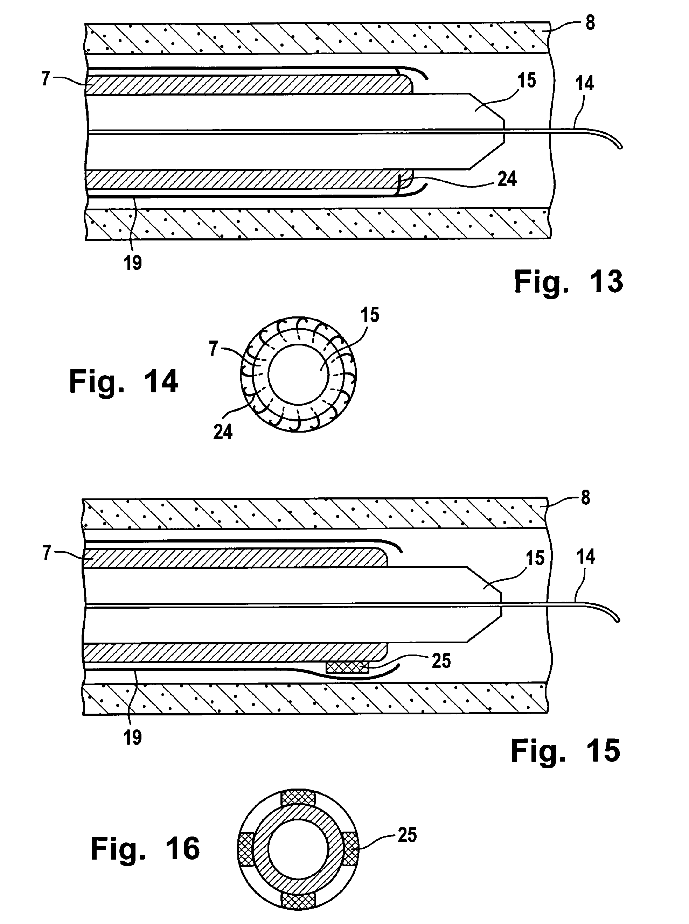

Bei den Ausführungsformen der Figuren 11 und 12 sind entsprechende federnde Drähte 23 im Konduit 7 gleitbar geführt und können nach Einsetzen des Konduits nach vorne geschoben werden, so dass sie aus dem Konduit 7 heraustreten und sich an der Wand 8 verankern.

Bei der Ausführungsform der Figur 13 und 14 sind kleine Häkchen 24 vorgesehen, die sich nach Entfernen der schlauchförmigen Hülse 19 an die Wand der Ader 8 anlegen. In Figur 13 ist dies im Querschnitt, in Figur 14 in Draufsicht auf das Ende des Konduits 7 gezeigt.

Bei der Ausführungsform der Figur 15 und 16 sind am Umfang am distalen Ende des Konduits 7 Elemente 25 aus einem Hämostyptikum vorgesehen, die nach Entfernen der Hülse 19 und nach Kontakt mit Blut aufquellen. Im Gegensatz zur Ausführungsform der Figur 7 lassen diese Elemente 25 aber zwischen sich Freiräume, so dass hier Blut zurückströmen kann.

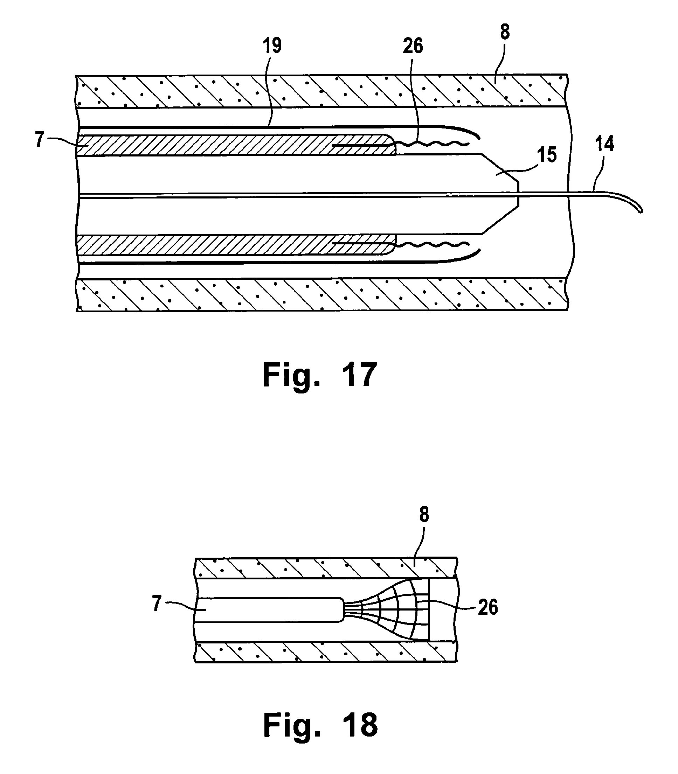

In den Figuren 17 und 18 sind schließlich Spreizfedern 26 gezeigt, die gemäß Figur 17 zunächst durch die schlauchförmige Hülse 19 zusammengehalten werden und sich dann nach Entfernen derselben, wie dies in Figur 18 gezeigt ist, aufspreizen und so das Konduit 7 an seinem distalen Ende an der Wand der Ader 8 verankern.

## Patentansprüche

1. In eine Ader (4, 8) des Blutkreislaufs einführbares Konduit (7), insbesondere zur Myokardrevaskularisation durch partielle Arterialisierung des Koronarsinus, **dadurch gekennzeichnet, dass** es am distalen Ende radial expandierbar ist.

2. Konduit (7) nach Anspruch 1, **dadurch gekennzeichnet, dass** am distalen Ende (13) des Konduits (7) im Lumen desselben ein aufweitbarer Stent (17) angeordnet ist.

3. Konduit (7) nach Anspruch 1, **dadurch gekennzeichnet, dass** das distale Ende (13) des Konduits (7) außen auf das Konduit (7) so zurückgefaltet ist, dass eine ringförmige Tasche gebildet ist, in der ein aufweitbarer Stent (17) angeordnet ist.

4. Konduit (7) nach Anspruch 2 oder 3, **dadurch gekennzeichnet, dass** der Stent (17) durch einen Ballonkatheter (18) aufweitbar ist.

5. Konduit (7) nach Anspruch 2 oder 3, **dadurch gekennzeichnet, dass** der Stent (17) selbstexpandierend ist.

6. Konduit (7) nach Anspruch 1, **dadurch gekennzeichnet, dass** das Konduit (7) am distalen Ende (13) an seiner äußeren Oberfläche mit einer ringförmigen Hülse (21) aus einem Hämostyptikum versehen ist.

7. Konduit (7) nach Anspruch 1, **dadurch gekennzeichnet, dass** im distalen Endes des Konduits (7) ein oder mehrere radial expandierbare Drahtelemente (22) angeordnet sind.

8. Konduit (7) nach Anspruch 1, **dadurch gekennzeichnet, dass** im distalen Ende (13) des Konduits (7) ein oder mehrere federnde Elemente (23, 24, 26) verankert sind, deren vorstehende Enden radial expandierbar sind.

9. Konduit (7) nach Anspruch 8, **dadurch gekennzeichnet, dass** die federnden Elemente (23) sich durch das Konduit (7) erstrecken und im demselben vom proximalen Ende her verschiebbar sind.

10. Konduit (7) nach Anspruch 8 oder 9, **dadurch gekennzeichnet, dass** die federnden Elemente hakenförmig (24) sind.

11. Konduit (7) nach Anspruch 8 oder 9, **dadurch gekennzeichnet, dass** die federnden Elemente Spreizfedern (26) sind.

12. Konduit (7) nach Anspruch 1, **dadurch gekennzeichnet, dass** das Konduit (7) am distalen Ende (13) an seiner äußeren Oberfläche mit mehreren am Umfang angeordneten Elementen (25) aus einem Hämostyptikum versehen ist.

13. Konduit (6) nach einem der Ansprüche 1 bis 12, **dadurch gekennzeichnet, dass** er an seinem distalen Ende einen Ballon zum Aufweiten des Konduits (7) und/oder der verankerungselemente aufweist.

14. Anordnung zum Einbringen eines Konduits (7), **dadurch gekennzeichnet, dass** sie einen Führungsdraht (14), ein Konduit (7) nach einem der Ansprüche 1 bis 13, einen Katheter (15), auf den das Konduit (7) aufgeschoben ist, und eine schlauchförmige Hülse (sheet 19) aufweist, der auf das Konduit (7) aufgeschoben und nach Einbringung des Konduit (7) nach proximal wenigstens teilweise zurückziehbar ist.
